# EUROPEAN PATENT APPLICATION

(11) **EP 4 046 643 A1**
(43) Date of publication of application: **24.08.2022**
(21) Application number: 21158813.2
(22) Date of filing: 23.02.2021
(51) Int. Cl.: A61K 35/741, A61K 35/745, A61P 35/00

(54) **A METHOD OF TREATING OR PREVENTING COLORECTAL CANCER**

(71) Applicant: University College Cork - National University of Ireland, Cork, Cork City (IE)
(72) Inventor: Santos Almeida, Ana, Cork (IE); Ghosh, Tarini, Cork (IE); Shanahan, Fergus, Cork (IE); O'Toole, Paul, Cork (IE)
(74) Representative: Purdylucey Intellectual Property

(57) **Abstract**

A composition comprising a pure culture of at least one bacterium and optionally a consortium of bacteria, **for use** in the treatment or prevention of colorectal cancer in a subject is described. The composition is administered to the colon of the subject, and the or each bacterium is a human gut-derived bacterium selected from: *Coprococcus comes; Ruminococcus lactaris; Bifidobacterium longum; Bifidobacterium pseudocatenulatum;* and *Bacteroides finegoldii.*

## Description

### Field of the Invention

The present invention relates to a method of treating or preventing colorectal cancer. In particular, the invention relates to a method of treating a subject with a colorectal tumour to inhibit or slow the growth of the tumour.

### Background to the Invention

Colorectal cancer (CRC) is among the top three causes of global cancer-related mortality and the incidence continues to increase worldwide. Risk factors for CRC include age, a diet low in fiber and rich in fat and red meat, and chronic inflammation of the gastrointestinal tract. All these factors are closely associated with altered composition and function of the gut microbiota. The gut microbiota from patients with polyps or CRC differs from that of healthy individuals. However, whether this "dysbiosis" contributes to disease or is a consequence of cancer is not yet clear. Differences in the gut microbiota also explain why some patients with cancer benefit from new cancer immunotherapies while other patients do not.

Studies to date in mice have indicated several ways by which specific bacterial species might impact CRC development. Proposed CRC-promoting bacteria or "oncogenicbacteria" include *Fusobacterium nucleatum,* genotoxic (pks+) *Escherichia coli, enterotoxigenic Bacteroides fragilis, Streptococcus gallolyticus,* and *Peptostreptococcus anaerobius.* Despite the increasing evidence implicating the gut microbiota in CRC development, the role of microbial community-driven pathogenicity as distinct from single taxa still needs to be elucidated.

Bacteria can directly contribute to the development of CRC by the release of genotoxic factors, production of carcinogenic metabolites, or by interacting with the immune system. Infiltration by immune cells heavily impacts clinical outcome in CRC. Numbers of intratumoral T cells correlate positively with better CRC outcomes, including disease-free and overall survival. However, the role of innate immune cells in CRC is less clear. Under certain circumstances, neutrophils and macrophages release radical oxygen species and nitrous oxide, which can potentially initiate a carcinogenic cascade by damaging the genomic integrity of colonic epithelial cells. The presence of specific immune cells in the tumor microenvironment, as well as the release of lymphocyte-attracting chemokines and cytokines, can be modulated by the microbiota, either directly or through their metabolites. Understanding how specific microbiota taxa and their metabolites differentially modulate the host immune response has important clinical implications for CRC patients, including diagnosis, and potentially also treatment.

In previous analyses of the gut microbiome in CRC patients, clustering methodologies were used to identify six co-abundance groups or CAGs (i.e. co-abundance associations of genera) (Flemer, B. et al. Tumour-associated and non-tumour-associated microbiota in colorectal cancer. Gut 66, 633-643 (2017*)),* which were subsequently simplified to five CAGs *(*Flemer, B. et al. The oral microbiota in colorectal cancer is distinctive and predictive. Gut 67, 1454-1463 (2018*).* A single subject harbored multiple CAGs but their relative abundance differed between CRC patients and healthy controls, with three of the 5 CAGs being more abundant in CRC patients. Tumor biopsies from patients whose microbiome was dominated by these CAGs showed differential expression of 18 genes involved in inflammation and CRC progression.

### Summary of the Invention

The Applicant employed human-into-mice fecal microbiota transplants from patients with adenomas or adenocarcinomas with a 'low risk' microbiota (in which the *Lachnospiraceae* CAG is dominant) or a 'high risk' microbiota (in which the Pathogen CAG is dominant), and showed that these differentially affected tumor growth and that distinct bacterial taxa correlated with tumorigenesis, different metabolic pathways, and divergent systemic immune responses that correlated with tumor volume. The Applicant has demonstrated that mammals colonized with bacterial strains of the *Lachnospiraceae* CAG, successfully attenuated or prevented the development of colorectal cancer, and in particular prevented tumour growth, or resulted in reduced tumour volume, compared with (a) mammals that did not receive the intervention and (b) mammals whose microbiome was composed of bacterial strains of the high risk microbiota in which the Pathogen CAG is dominant. The invention therefore broadly relates to the use of strains of bacteria of the *Lachnospiraceae* CAG, and the use of agents to reduce the abundance of bacterial strains of the Pathogen CAG in the microbiota of a subject, in the treatment and prevention of colorectal cancer in a mammal.

In a first aspect, the invention relates to the use of a bacterium in the treatment or prevention of colorectal cancer in a subject, the method comprising administering the bacterium to the colon of the subject, in which the bacterium is selected from: *Coprococcus comes; Ruminococcus lactaris; Bifidobacterium longum; Bifidobacterium pseudocatenulatum;* and *Bacteroides finegoldii.*

In any embodiment, a consortium of bacteria is administered to the subject, in which the consortium of bacteria includes at least one species selected from: *Coprococcus comes; Ruminococcus lactaris; Bifidobacterium longum; Bifidobacterium pseudocatenulatum;* and *Bacteroides finegoldii.*

In a further aspect, the invention provides a composition comprising a bacterium, typically a consortium of bacteria, including at least one species selected from: *Coprococcus comes; Ruminococcus lactaris; Bifidobacterium longum; Bifidobacterium pseudocatenulatum;* and *Bacteroides finegoldii,*

In any embodiment, the composition is a food, beverage or nutritional supplement.

In any embodiment, the composition is a pharmaceutical composition and comprises a suitable pharmaceutical excipient.

In any embodiment, the or each bacterium is provided as a pure culture of the bacterium.

In any embodiment, the consortium of bacteria includes at least two species of bacteria selected from: *Coprococcus comes; Ruminococcus lactaris; Bifidobacterium longum; Bifidobacterium pseudocatenulatum;* and *Bacteroides finegoldii.*

In any embodiment, the consortium of bacteria includes at least three species of bacteria selected from: *Coprococcus comes; Ruminococcus lactaris; Bifidobacterium longum; Bifidobacterium pseudocatenulatum;* and *Bacteroides finegoldii.*

In any embodiment, the consortium of bacteria includes at least four species of bacteria selected from: *Coprococcus comes; Ruminococcus lactaris; Bifidobacterium longum; Bifidobacterium pseudocatenulatum;* and *Bacteroides finegoldii.*

In any embodiment, the consortium of bacteria includes at least five species of bacteria selected from: *Coprococcus comes; Ruminococcus lactaris; Bifidobacterium longum; Bifidobacterium pseudocatenulatum;* and *Bacteroides finegoldii.*

In any embodiment, the consortium of bacteria includes: *Coprococcus comes; Ruminococcus lactaris; Bifidobacterium longum; Bifidobacterium pseudocatenulatum;* and *Bacteroides finegoldii.*

In any embodiment, the consortium of bacteria includes *Coprococcus comes.*

In any embodiment, the consortium of bacteria includes *Ruminococcus lactaris.*

In any embodiment, the consortium of bacteria includes *Bifidobacterium longum.*

In any embodiment, the consortium of bacteria includes *Bifidobacterium pseudocatenulatum.*

In any embodiment, the consortium of bacteria includes *Bacteroides finegoldii.*

In any embodiment, the species of bacteria is a human gut-derived strain of bacteria.

In any embodiment, substantially all of the species of bacteria in the consortium of bacteria are human gut-derived strains of bacteria.

In any embodiment, the species of bacteria, or some or all of the species in the consortium of bacteria) is derived from the gut of a subject with a microbiota (e.g. mucosal microbiota) dominated with a *Lachnospiraceae* CAG (co-abundance association of genera). The *Lachnospiraceae* CAG is described in Flemer, B. et al. (The oral microbiota in colorectal cancer is distinctive and predictive. Gut 67, 574 1454-1463 (2018)).

In any embodiment, the consortium of bacteria is administered orally in an oral dosage form configured for gastric transit and release in the gastrointestinal tract distal of the stomach, for example the ileum or colon.

In any embodiment, the consortium of bacteria is administered rectally.

The following strains may be used in the methods and compositions of the invention:
*Coprococcus comes* ATCC27758
*Coprococcus comes* SL7/1
*Bacteroides finegoldii* DSM 17565
*Bacteroides finegoldii* DSM 108067
*Bacteroides finegoldii* JCM 13346
*Bacteroides finegoldii* CCUG 68636
*Bifidobacterium longum* APC 1477
*Bifidobacterium longum* DPC 6323
*Bifidobacterium longum* 1941
*Bifidobacterium pseudocatenulatum* LI09
*Bifidobacterium pseudocatenulatum* H7-2

In any embodiment, the bacterium or consortium of bacteria is administered as a once-off treatment.

In any embodiment, the bacterium or consortium of bacteria is administered at least once per week over a treatment period of at least 4 weeks, and preferably at least 5, 6, 7, 8, 9, 10, 11, 12, 14, 16, 18 or 20 week period.

In any embodiment, about 10³ to 10¹² cfu, or about 10⁴ to 10¹² cfu, or about 10⁶ to 10¹⁰ cfu of the bacterium, or of each bacterium in the consortium, is administered to the subject.

In any embodiment, the pharmaceutical composition comprises about 10³ to 10¹² cfu, or about 10⁴ to 10¹² cfu, or about 10⁶ to 10¹⁰ cfu of the bacterium, or of each bacterium in the consortium.

In any embodiment, the pharmaceutical composition is an oral dosage form.

In any embodiment, the pharmaceutical composition comprises bacteria encapsulated in a shell or matrix configured for gastric transit and release in the gut distal of the stomach.

In any embodiment, a prebiotic is co-administered with the bacteria or consortium of bacteria. In any embodiment, the composition comprises a prebiotic.

In any embodiment, the bacteria, consortium of bacteria (or pure cultures) comprise live bacteria.

In any embodiment, the bacteria, consortium of bacteria (or pure cultures) are freeze-dried.

In any embodiment, the method additionally comprises administering an anti-microbial agent targeted against a bacterium selected from the group consisting of:
*Clostridium hathewayi;*
*Flavonifractor plautii;*
*Bacteroides fragilis;*
*Bacteroides salyersaie;* and
*Clostridium bolteae.*

In a third aspect, the invention relates to the use of an anti-microbial composition in the treatment of prevention of colorectal cancer in a subject, the method comprising administering the anti-microbial composition to the colon of the subject, in which the anti-microbial composition comprises an anti-microbial agent that is effective against a bacterium selected from: *Clostridium hathewayi; Flavonifractor plautii; Bacteroides fragilis; Bacteroides salyersaie;* and *Clostridium bolteae.*

In a fourth aspect, the invention provides a pharmaceutical composition comprising an anti-microbial agent that is effective against a bacterium selected from: *Clostridium hathewayi; Flavonifractor plautii; Bacteroides fragilis; Bacteroides salyersaie;* and *Clostridium bolteae,* in combination with a suitable pharmaceutical excipient.

In any embodiment, the anti-microbial agent is effective against a plurality of bacteria (for example, two, three, four or five) selected from: *Clostridium hathewayi; Flavonifractor plautii; Bacteroides fragilis; Bacteroides salyersaie;* and *Clostridium bolteae.*

In any embodiment, the anti-microbial composition comprises a plurality of anti-microbial agents (for example, two, three, four or five) each of which is effective against a different bacterium selected from: *Clostridium hathewayi; Flavonifractor plautii; Bacteroides fragilis; Bacteroides salyersaie;* and *Clostridium bolteae.*

In any embodiment, the anti-microbial is a pathogen-specific antimicrobial agent.

In any embodiment, the anti-microbial agent is engineered de novo through membrane-protein biomimicry. Method of making such anti-microbials is described in Simonsen et al. (Nature Biomedical Engineering (4 January 2021)).

Anti-microbial agents that selectively target specific pathogens are described in Seal et al. (Veterinary Research volume 49, Article number: 66 (2018)), and Xiao et al. (ACS Nano, 2019 Feb 26;13(2):1511-1525).

Examples of anti-microbial agents that selectively target the Pathogen CAG, specifically *Clostridium hathewayi; Flavonifractor plautii; Bacteroides fragilis; Bacteroides salyersiae;* and *Clostridium bolteae.* include anti-microbial peptides and bacteriophages *(*Federici, S., Nobs, S. P. & Elinav, E. Phages and their potential to modulate the microbiome and immunity. Cell Mol Immunol 1-16 (2020) doi:10.1038/s41423-020-00532-4*.)*

In any embodiment, the method additionally comprises administering a bacterium to the colon of the subject, in which the bacterium is selected from: *Coprococcus comes; Ruminococcus lactaris; Bifidobacterium longum; Bifidobacterium pseudocatenulatum;* and *Bacteroides finegoldii.*

In any embodiment, a consortium of bacteria is administered to the subject, in which the consortium of bacteria includes at least one (or two, three, four, five or six) species selected from: *Coprococcus comes; Ruminococcus lactaris; Bifidobacterium longum; Bifidobacterium pseudocatenulatum;* and *Bacteroides finegoldii.*

In any embodiment, the method is a method of treatment of a subject with a solid colorectal tumor.

In any embodiment, the method is a method of inhibiting or slowing the growth of a solid colorectal tumor in a subject.

In any embodiment, the subject has an adenoma and the method is a method of preventing, slowing or inhibiting development of an adenocarcinoma in the subject.

In any embodiment, the subject exhibits one or more symptoms of colorectal cancer.

In any embodiment, the subject has a family history of colorectal cancer.

In any embodiment, the subject had had surgery to resect a cancerous section of the colon or rectum.

In a further aspect, the invention relates to a method of formulating a pharmaceutical composition comprising the steps of:
isolating at least one strain of the *Lachnospiraceae* CAG (typically from a fecal or tumour sample from a subject), which in one embodiment is selected from *Coprococcus comes; Ruminococcus lactaris; Bifidobacterium longum; Bifidobacterium pseudocatenulatum;* and *Bacteroides finegoldii;*
culturing the isolated strain to provide a pure culture of the strain; and
combining the pure culture of the strain with a pharmaceutical excipient to provide the pharmaceutical composition.

In any embodiment, the method comprises:
isolating a plurality of strains of the *Lachnospiraceae* CAG (for example two, three, four, five or six) (in one embodiment the strains are selected from *Coprococcus comes; Ruminococcus lactaris; Bifidobacterium longum; Bifidobacterium pseudocatenulatum;* and *Bacteroides finegoldii;*
culturing the isolated strains to provide a pure culture of each strain; and
combining the pure cultures with a pharmaceutical excipient to provide the pharmaceutical composition.

In one embodiment, the method comprises identifying a subject with a microbiome (ideally a mucosal microbiota) dominated with a *Lachnospiraceae* CAG, and isolating the or each strain from the subject, typically from a faecal or CRC tumour sample obtained from the subject;

In any embodiment, the pure cultures comprise live bacteria.

In any embodiment, the pure cultures are freeze-dried.

In any embodiment, a prebiotic is added to the composition.

In any embodiment, the subject does not have colorectal cancer.

In any embodiment, the subject has an adenoma and no adenocarcinoma.

In a further aspect, the invention relates to a fecal sample from a donor with a microbiome (ideally a mucosal microbiota) dominated with a *Lachnospiraceae* CAG; for use in the treatment or prevention of a colorectal cancer in a subject, in which the fecal sample is administered to the colon of the subject.

In any embodiment, the subject does not have colorectal cancer.

In any embodiment, the subject has an adenoma and no adenocarcinoma.

Other aspects and preferred embodiments of the invention are defined and described in the other claims set out below.

### Brief Description of the Figures

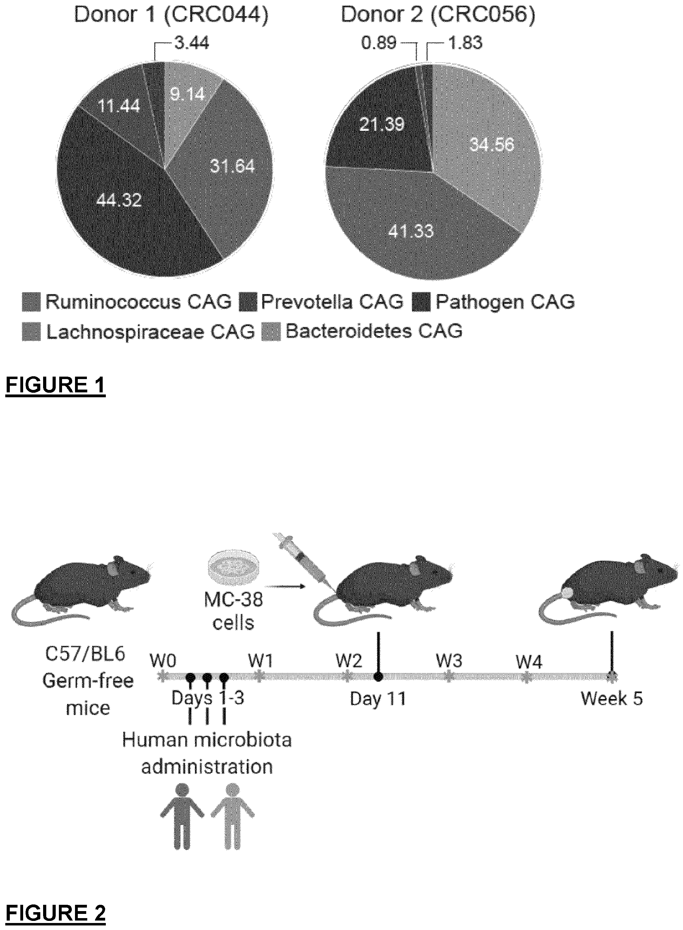

**FIG.** 1. Validation of microbiota composition in human donors for the pre-clinical mouse trial. Colon resections were collected at surgery and the mucosal microbiota was profiled using 16S rRNA sequencing. Pie-charts represent the abundance of the five bacterial CAGs on adenoma or tumor samples from each donor. Donor 1 (CRC044), diagnosed with a T3N2 rectum adenocarcinoma, was selected based on the high abundance of a Pathogen CAG microbiota and Donor 2 (CRC056), diagnosed with a tubulovillous adenoma, was selected based on the high abundance of a *Lachnospiraceae* CAG microbiota.
**FIG.2** Experimental design of the pre-clinical trial with a humanized MC-38 model of CRC.

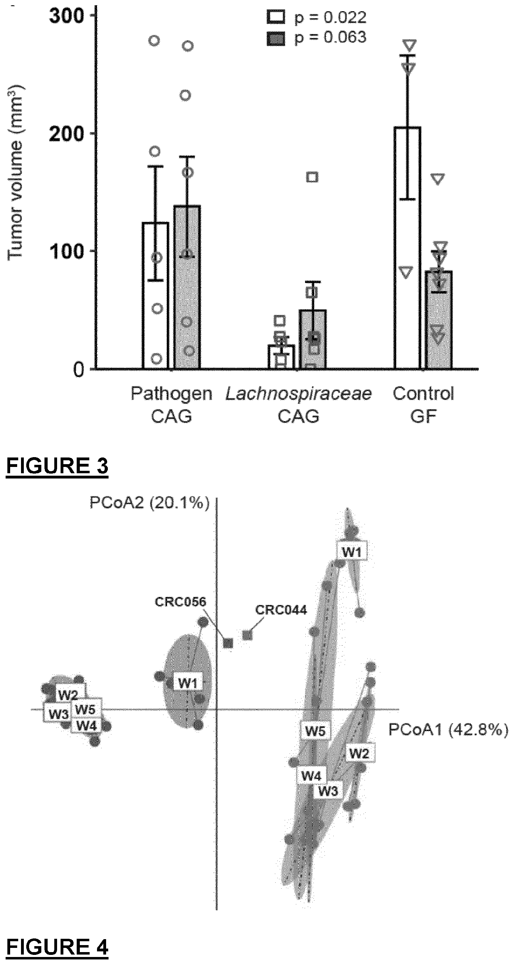

**FIG. 3** Tumor growth is reduced in mice receiving the *Lachnospiraceae* microbiota compared to mice receiving the Pathogen CAG microbiota or control germ-free (GF) mice. Tumor volume was measured with a caliper at endpoint and calculated as (length x width2)/2. Overall p values were calculated with the Kruskal-Wallis test. Data indicate mean ±SEM. N=3-6 biological replicates/group per condition. Data from two independent experiments are shown by open and grey bars.
**Fig. 4****:** Relatedness (β-diversity) of the fecal microbiota of the two human donors and respective transplanted mice at different time-points represented by principal coordinate analysis (PCoA) on Bray-Curtis distance matrix (PERMANOVA r2=0.79; p value = 0.001). W, week.

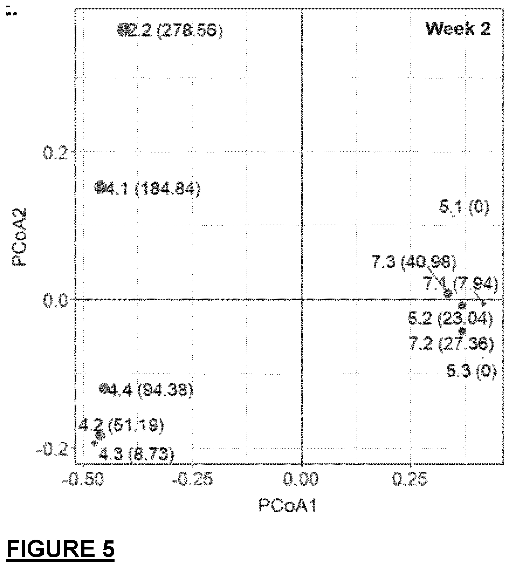

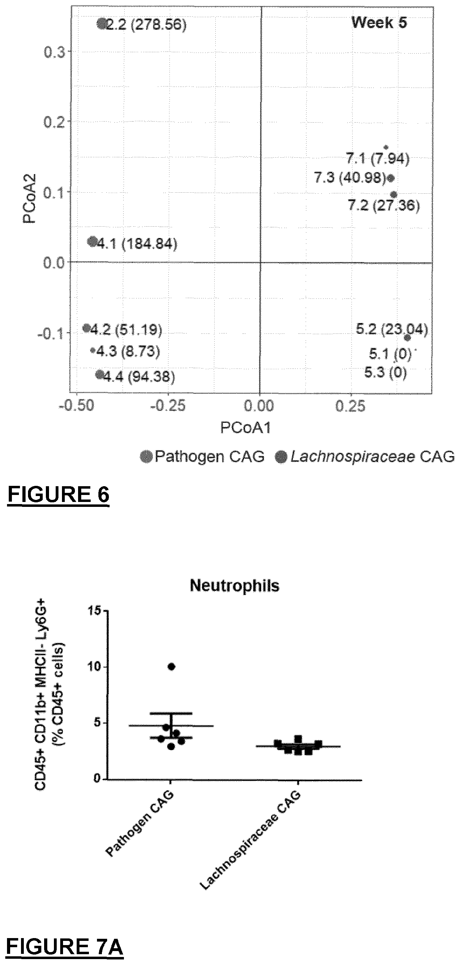

**Figs. 5** **and** **6****:** PCoA plots of the Metaphlan2 species-level taxa profiles of the murine fecal microbiomes, performed at week 2 (Fig. 5) and week 5 (Fig. 6). Fecal microbiome profiles corresponding to the Pathogen CAG donor and the *Lachnospiraceae* CAG donor are colored in red and green, respectively. Each point corresponds to a specific mouse ID and the corresponding tumor volume is shown within parentheses. The size of each point is proportional to the tumor volume. PERMANOVA r2=0.24; p = 0.016 (week 2), r2=0.25; p value = 0.006 (week 5).

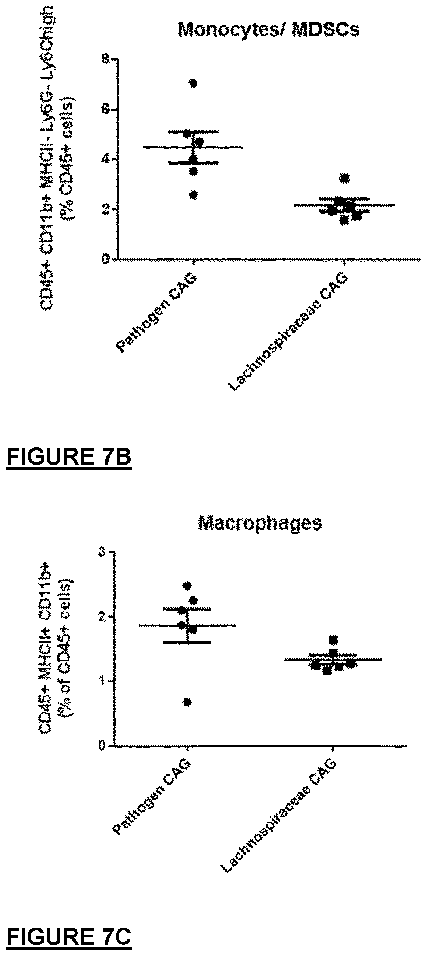

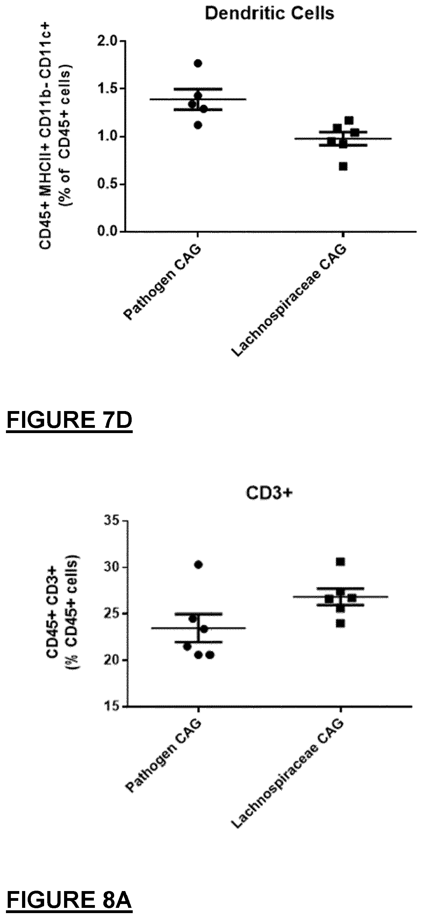

**Fig. 7****:** Spleens from mice with the Pathogen CAG have more neutrophils **(****Fig. 7A** - CD45+CD11b+MHCII-Ly6G+Ly6low), monocytes **(****Fig. 7B** - CD45+ CD11b+ MHCII- Ly6G-LyChigh), macrophages **(****Fig. 7C** - CD45+CD11b+MHCII+), and dendritic cells **(****Fig. 7D** - CD45+ MHCII+ CD11b- CD11c+), as determined by flow cytometry gated on CD45+ cells. Panels show quantification of neutrophils, monocytes, macrophages, and dendritic cells.

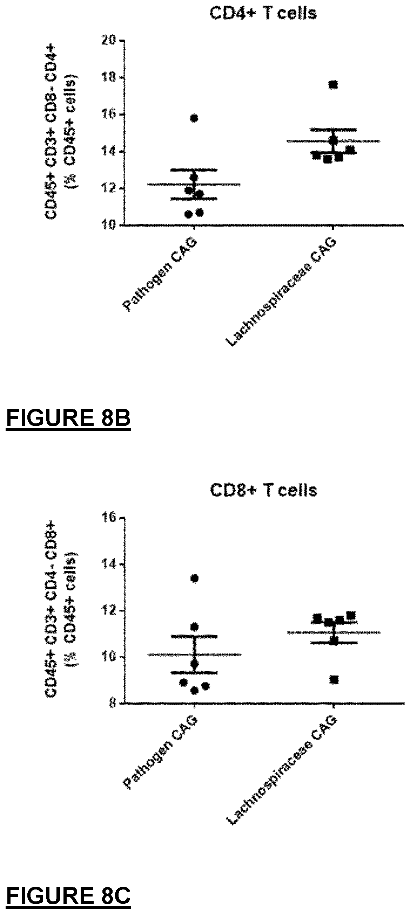

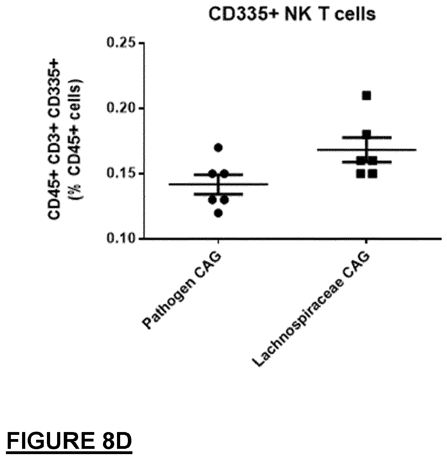

**Fig. 8****:** Spleens from mice with the *Lachnospiraceae* CAG have more CD3+ **(****Fig. 8A****),** CD4+ **(****Fig. 8B****),** CD8+ **(****Fig. 8C****),** and NK T cells **(****Fig. 8D****),** as determined by flow cytometry gated on CD45+ cells. P values were determined by Mann-Whitney U test and are represented in each plot. Data indicate mean ±SEM. n = 6 biological replicates/group.

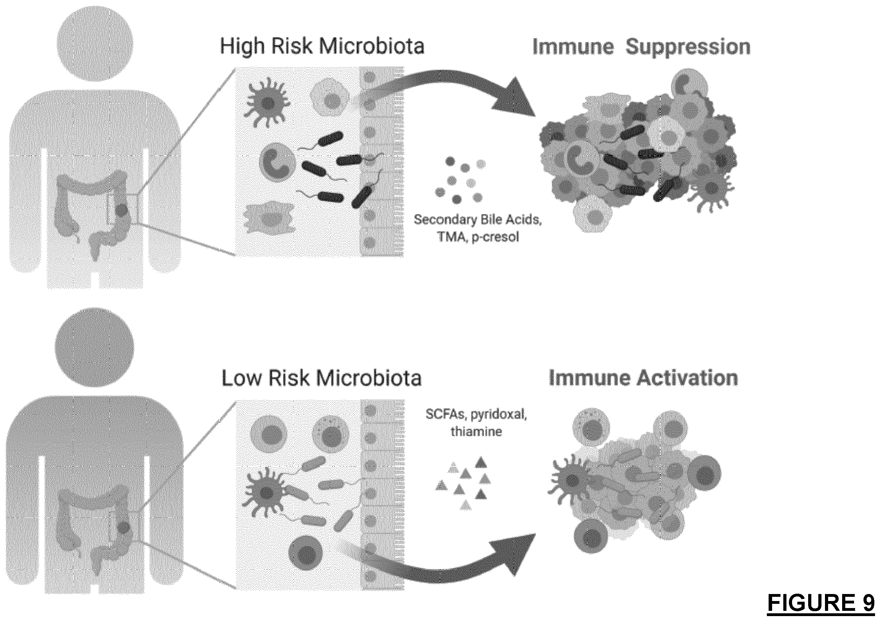

Fig. 9: Model of how the high-risk Pathogen and low-risk *Lachnospiraceae* CAGs differentially modulate the tumor immune response (Created with BioRender.com).

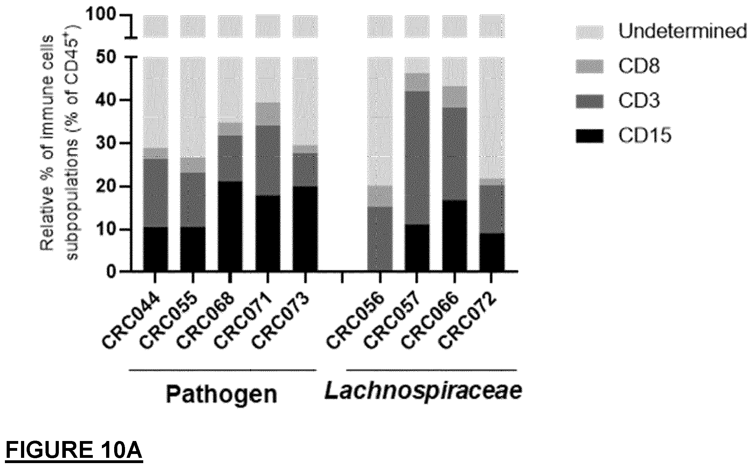

**Fig 10A****:** Quantification of immune infiltrate (CD45⁺), T-cell infiltrate (CD3⁺ and CD8⁺) and neutrophil infiltrate (CD15⁺) in human CRC biopsies from Pathogen (n=5) and Lachnospiraceae-enriched tumors (n=4). 2 sections/tumor and 3 ROIs quantified per section, means are shown, group comparison with one-way ANOVA.

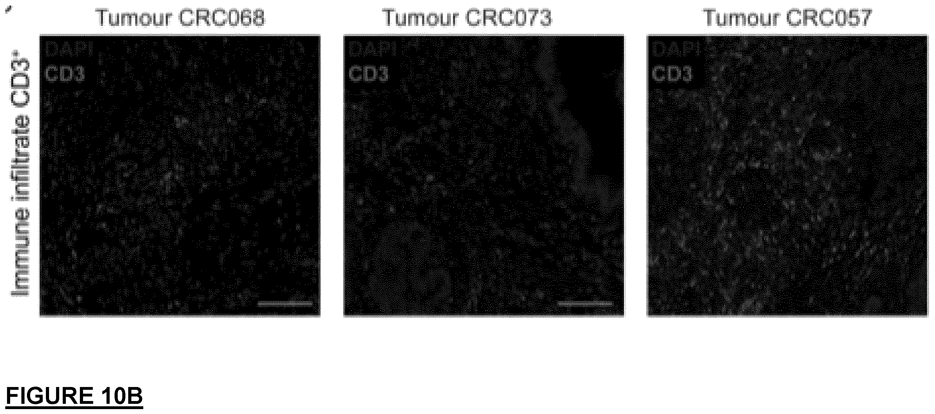

**Fig. 10B****:** Immunofluorescence representative images of Pathogen CAG-enriched tumors (CRC068 and CRC073) and *Lachnospiraceae* CAG-enriched tumors (CRC057) human tumors showing that more CD3⁺ T cells (red) infiltrate into *Lachnospiraceae* tumors, while more CD15⁺ neutrophils (colour) infiltrate into Pathogen tumors. Counterstained with nuclear dye DAPI. All tumors were analyzed and for each tumour 3 ROIs were quantified per section (n=2 sections/tumour/staining). Scale bars 100µm.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis". Thus, the methods and compositions described herein may be applied to treating a subject with a colorectal cancer to inhibit or slow the progression of the disease, to slow the spreading of the disease, to slow tumour growth, to reduce tumour volume, to reduce the occurrence of symptoms of the disease. The methods and compositions may also be applied to a subject with a benign colorectal tumour (or a subject at risk of developing colorectal cancer as a result of genotypic or phenotypic factors) to prevent or slow the development of the disease. The methods may also be applied to a subject with a benign colorectal tumour (or a subject at risk of developing colorectal cancer as a result of genotypic or phenotypic factors) to prevent or slow the development of the disease. The methods may also be applied to healthy subjects.

As used herein, an effective amount or a therapeutically effective amount of an agent (i.e. bacterium or composition of the invention) defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

As used herein, the term *"Lachnospiraceae* CAG" refers to the group of taxa identified in Flemer *et* a/2017 (Flemer B, Lynch DB, Brown JM, Jeffery IB, Ryan FJ, Claesson MJ, O'Riordain M, Shanahan F, O'Toole PW. Tumour-associated and non-tumour-associated microbiota in colorectal cancer. Gut. 2017 Apr;66(4):633-643) and further refined in Flemer et al 2018 (Flemer B, Lynch DB, Brown JM, Jeffery IB, Ryan FJ, Claesson MJ, O'Riordain M, Shanahan F, O'Toole PW. Tumour-associated and non-tumour-associated microbiota in colorectal cancer. Gut. 2017 Apr;66(4):633-643), that were enriched for members belonging to the *Lachnospiraceae* CAG and were negatively associated with the CRC-associated pathobionts.

As used herein, the term "Pathogen CAG" refers to the group of taxa identified in Flemer *et al* 2017 (Flemer B, Lynch DB, Brown JM, Jeffery IB, Ryan FJ, Claesson MJ, O'Riordain M, Shanahan F, O'Toole PW. Tumour-associated and non-tumour-associated microbiota in colorectal cancer. Gut. 2017 Apr;66(4):633-643) and further refined in Flemer et al 2018 (Flemer B, Lynch DB, Brown JM, Jeffery IB, Ryan FJ, Claesson MJ, O'Riordain M, Shanahan F, O'Toole PW. Tumour-associated and non-tumour-associated microbiota in colorectal cancer. Gut. 2017 Apr;66(4):633-643), that contained multiple CRC-associated pathobionts.

As used herein, the term "pure culture" as applied to a specific strain refers to culture of bacteria containing only the specific strain. Methods of preparing pure cultures of bacteria are well known to a person skilled in the art and generally involve transferring a small sample of a mixed culture into new, sterile growth medium in such a manner as to disperse the individual cells across the medium surface or by thinning the sample manyfold before inoculating the new medium. Both methods separate the individual cells so that, when they multiply, each will form a discrete colony, which may then be used to inoculate more medium, with the assurance that only one type of organism will be present. Isolation of a pure culture may be enhanced by providing a mixed inoculum with a medium favouring the growth of one organism to the exclusion of others.

As used herein, the term "pharmaceutical composition" refers to a therapeutically effective amount of the strain of the invention, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. In the case of the present invention, the term "therapeutically effective amount" should be taken to mean an amount of therapeutic which results in a clinically significant increase in proliferation of target cells, for example gut epithelial cells or skin epithelial cells.

As used herein, the term "adjuvant" means an agent that enhances the recipient's immune response to an immunogenic peptide or protein. Details of suitable adjuvant compositions are well known to those skilled in the art.

As used herein, the term "pharmaceutically acceptable carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Pharmaceutical compositions formulated or configured for oral administration and gastric transit are known in the art and include those described below:
- Sathish et al (Int. J. Pharm. Sci.2013; 258-269);
- Kushal et al (Int. Res. J. Pharm. 2013, 4(3));
- Philip et al (Oman Med J. 2010, 25(2));
- Polymers for controlled drug delivery - Peter Tarcha (CRC Press, 21 November 1990);
- Pharmaceutical coating technology - Michael Aulton et al (Taylor & Francis 27 October 1995);
- http://www.slideshare.net/Balimusale/oral-controlled-drug-delivery-system;
- European Patent No: 2418968 (Teagasc); and
- European Patent No: 2097072 (RCSI).
- Brayden et al. (European Journal of Pharmaceutical Sciences 79 (2015), 102-111.
- Tambuwala et al. (Journal of Controlled Release 217 (2015) 221-227.
- Zhang et al. Evaluation of alginate-whey protein microcapsules for intestinal delivery of lipophilic compounds in pigs (J. Sci. Food Agric. (2015).
- Lamprecht et al. (Journal of Controlled Release 104 (2005) 337-346.
- Hua et al. (Nanomedicine: Nanotechnology, Biology and Medicine 11 (2015) 1117-1132.
- Drug Delivery: Fundamentals and Applications (Chapter 7, Oral Drug Delivery, Hillary and Brayden)

As used herein, the term "food" refers to a man-made food product including beverages, food additives and food supplements. Examples of foods include dairy products such as milk, yoghurt, cheese, cheese food, dairy powders, probiotic formulations, infant formula powders, follow-on milk formula, food for special medicinal purposes, meat products, soups, vegetable products, fruit juices, fruit products, breads, confectionary, cakes, sports supplements, nutritional supplements and the like.

The strain, consortium of strains or composition of the invention may be administered at least one per week over at treatment period of at least 4 weeks, and preferably at least 5, 6, 7, 8, 9, 10, 11, 12, 14, 16, 18 or 20 week period. Preferably, the strain or composition is administered several times a week, and ideally once a day. Compositions of the invention generally comprise between 10³ and 10¹² cfu of the strain of the invention per gram of dry weight of the composition. In one embodiment, the composition comprises10³ and 10¹² cfu, or 10⁴ and 10¹² cfu, or 10⁶ and 10¹⁰ cfu of the strain of the invention per gram of dry weight of the composition. A daily dose generally comprises between 10³ and 10¹² cfu of the strain.

As used herein, the term "administered to the colon" or "administered to the distal colon" should be understood to mean administered in such as way that the strain or consortium of strains or composition is delivered to the colon or distal colon. Forms of administration include oral administration and rectal administration via an enema.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Experimental

### Patients cohort and sample collection

All clinical studies were conducted after informed consent of the patients, following the guidelines of the Declaration of Helsinki. Ethical approval for this study was granted by the local Clinical Research Ethics Committee of the University College Cork, under the study number APC033. Patients' data were anonymized and stored under European Union General Data Protection Regulation.

A total of 32 treatment-naïve patients with adenomas or CRC were included in this study. Exclusion criteria included a personal history of CRC, inflammatory bowel disease (IBD), or inflammatory bowel syndrome (IBS), as well as chemotherapy or radiation therapy treatments. Individuals were not treated with antibiotics in the month prior to surgery but were administered antibiotics intravenously during surgery. Dietary data were collected using a validated Food Frequency Questionnaire (FFQ). Control subjects (i.e. routine colonoscopy) included individuals without a history of CRC, IBD or IBS, or antibiotic usage within 3 months, described elsewhere.

Resection samples were collected from patients undergoing colon surgery at Mercy University Hospital, Cork. Samples were collected from the tumor site (ON), 2-3 cm far from the tumor margin (OFF), and paired healthy tissue (approximately 10 cm from the tumor site). Samples collected were rapidly preserved in 1) RNA later for sequencing purposes; 2) methacarn for histology and 3) snap-frozen for subsequent analysis. Bowel preparations before surgery or colonoscopy were determined by the surgeon.

### DNA and RNA extraction of human biopsies

Human colon resections were placed in RNAlater (Qiagen) at the time of resection, stored at 4°C for 12 h, and then stored at -20°C until processing. Genomic DNA and total RNA were extracted using the AllPrep DNA/RNA kit (Qiagen). For tissue samples, ∼20 mg of tissue was placed into bead tubes containing 250 µl of 0.1 mm sterile glass beads (Biospec Products) and three 3-4 mm sterile glass beads (Biospec Products). Next, 600 mL of buffer RLT (Qiagen, USA) containing 1% β-mercaptoethanol was added and the sample was homogenized in a MagnaLyzer (Roche) for two pulses of 15 s each at full speed. In between the homogenization steps, the samples were put on ice for 30 s. The extraction was then carried out using the AllPrep DNA/RNA extraction kit (Qiagen), following the manufacturer's instructions. Genomic DNA was quantified using the Nanodrop 1000 (Thermo Scientific) and total RNA was quantified using the Bioanalyser (Agilent).

### Human fecal inoculum preparation

Two human donors were selected from the CRC patients' cohort based on their mucosal microbiota compositional profiles. Donor 1 (CRC044) is a female, 66 years old, diagnosed with a T3N2b rectum adenocarcinoma; donor 2 (CRC056) is a male, 65 years old, diagnosed with a tubulovillous adenoma with low-grade dysplasia (Figure 1). Stool samples were transferred to an anaerobic chamber within 1 min of voiding, transported to the lab in anaerobic bags, and transferred to an anaerobic hood in less than an hour. Fresh stools were diluted (ratio 1:10) in sterile pre-reduced PBS with 20% glycerol, before being aliquoted in 1 ml volumes in cryovials and stored at - 80°C until further use.

### DNA extraction of human fecal samples

Genomic DNA was extracted from fecal samples following the Repeated Beat Beating (RBB) Method, with the following modifications. Samples (0.25 g) were placed in sterile tubes containing one 3.5 mm zirconia bead and one scoop of 0.1 mm and 1 mm beads, respectively (Thistle Scientific). Fecal samples were homogenized via bead beating for 60 seconds (Mini-Beadbeater^{™}, BioSpec Products), with the samples cooled on ice for 60 seconds before another 60 seconds bead beating. Samples were then incubated at 70°C for 15 min and centrifuged at full speed for 5 min. Pooled supernatants were incubated with 350 ml of 7.5 M ammonium acetate (Sigma) and incubate on ice for 5 min. The remaining steps of DNA purification were performed using QIAamp columns (Qiagen). Genomic DNA was quantified using the Nanodrop 1000 (Thermo Scientific). Extracted genomic DNA was stored at -20°C until amplification.

### MC-38 cells culture

Murine C57BL/6 MC-38 colorectal tumor cells were obtained from Kerafast (ENH204-FP) and maintained in 5% CO2 at 37°C in Dulbecco's modified MEM (DMEM) medium supplemented with 10% heat-inactivated fetal bovine serum (Sigma), 2mM L-glutamine, 0.1 mM nonessential amino acids, 1 mM sodium pyruvate, 10mM HEPES and 100 units/ml penicillin/streptomycin antibiotic solution (all reagents from Gibco-Invitrogen). Cells were tested for Mycoplasma contamination every 4 - 6 weeks and before each experiment (Mycoalert Mycoplasma Detection kit, Lonza).

### Germ-free MC-38 mouse model

All animal protocols were approved by the Animal Experimentation Ethics Committee at University College Cork (UCC) and by the Health Products Regulatory Authority (HPRA) of Ireland, per EU Directive 2010/63/EU (HPRA Project authorization number AE19130/P055).

Germ-free (GF) mice were bred and maintained at the APC Germ-Free facility in dedicated axenic isolators (Bell Isolation Systems). Germ-free status was routinely monitored by culture-dependent and independent methods. Age-matched male C57BL/6 GF mice, 6-10 weeks of age, were group-housed 3-4 and transferred into sterile individual ventilated cages (IVCs) (Arrowmight, Hereford, UK) before undergoing human microbiota administration. Mice were kept in a 12-hour light-dark cycle and on ad libitum diet RM1 (autoclaved) (Special Diet Services, #0103). Water and diet were batched at the beginning of the experiment to exclude possible variations between batches. A schematic overview of the experimental study design is presented in Figure 2. Animals were pipette-dosed with 100 µl of fecal slurry or control PBS, per day for 3 consecutive days. Groups were as follows: "Pathogen CAG" group, inoculated with fecal slurry from donor 1 (patient CRC044); "Lachnospiraceae CAG" group, inoculated with fecal slurry from donor 2 (patient CRC056); and, GF control group inoculated with 100 µl of reduced PBS + glycerol 20%. After microbiota administration and colonization, MC-38 cells were orthotopically injected into the rectal submucosa, as previously described (Zigmond, E. et al. Utilization of murine colonoscopy for orthotopic implantation of colorectal cancer. PloS one 6, e28858 (2011*)).* Briefly, mice were anesthetised using a mixture of Ketamine/Medetomidine (75 mg/kg ketamine (100mg/ml), 0.5 mg/kg medetomidine (1mg/ml) subcutaneously. Injection of 20 µl of 5x105 (trial 1) or 1x105 (trial 2) MC-38 cancer cells was performed using an insulin syringe on the right flank. Fecal and blood samples were collected from each animal at various time points (weeks 0 to 5) as indicated in Figure 2. Two to three fecal pellets were collected at each time point and were immediately frozen in dry ice before being transferred to -80°C. Tumor size was measured by caliper at endpoint (week 4 in trial 1 and week 5 in trial 2), and tumor volume was calculated as (length x width2)/2.

### Genomic DNA extraction and microbiota profiling of murine fecal samples

Total DNA was extracted from mouse fecal samples using QIAamp DNeasy Blood and Tissue Kit (Qiagen) according to the manufacturer's protocol. The samples were placed in sterile tubes containing 0.1, 0.5, and 1.0 mm zirconia/glass beads (Thistle Scientific). 750 µl of IhibitEX Buffer (Qiagen) were added to the samples and then homogenized via bead beating for 1 minute (Mini-Beadbeater^{™}, BioSpec Products), with the samples cooled on ice for 1 minute followed by 40 seconds bead beating, and then heated at 95°C for 5 min. The subsequent steps of the DNA extraction were carried out as described in the Qiagen protocol using 200 µl of lysate, 15 µl of Proteinase K, and 200 µl of AL buffer and then incubated at 70 °C for 10 min. The genomic DNA was eluted in 50 µl elution buffer. Genomic DNA was quantified using the Nanodrop 1000 (Thermo Scientific,). Extracted genomic DNA was stored at -20°C until amplification.

Sequencing, Taxonomic and Functional Profiling
16S rRNA Gene Amplicon Sequencing: The V3-V4 region of the 16S rRNA gene was amplified, sequenced, and analyzed as described before *(*Ntemiri, A. et al. Retention of Microbiota Diversity by Lactose-Free Milk in a Mouse Model of Elderly Gut Microbiota. J Agr Food Chem 67, 2098-2112 (2019*)).* Amplification was performed with the universal 16S rRNA gene primer pair S-D-Bact-0341-b-S-17 and S-D-Bact-0785-a-A-2160. The Phusion High-Fidelity PCR Master Mix (ThermoFisher Scientific) was used for the amplification. The sequencing library was prepared using the Nextera XT V.2 Index Kit (Sets A and D, Illumina) according to the Illumina 16S MiSeq Sequencing Library protocol. The PCR products were purified with the SPRIselect reagent kit (Beckman Coulter, Inc.,). Amplicons were quantified with a Qubit dsDNA HS Assay Kit (Thermo Fischer Scientific) and pooled at the same concentration. Sequencing was performed on an Illumina MiSeq Platform (2x250 bp reads for human samples and 2x300 bp reads for mouse samples) by the Teagasc Next Generation DNA Sequencing Facility (Fermoy, Ireland).

Microbiota composition analysis of 16 S rRNA amplicon sequencing data: Primers were removed from raw sequences using Trimmomatic (v0.36). Paired-end sequencing reads (2x300bp) were joined using FLASH (v1.2.8). Demultiplexing and quality filtering were performed using the QIIME package (v1.9.1). The USEARCH sequence analysis tool (v8.1.186) was used for further quality filtering and de novo operational taxonomic unit (OTU) clustering. The sequences were filtered by length and sorted by size, and single unique sequences were removed. The remaining sequences were clustered into OTUs based on 97% identity. Subsequently, chimeras were removed with UCHIME, using the GOLD reference database. The original quality-filtered sequences were mapped against the OTUs (97% identity). OTU representative sequences were classified with 80% confidence threshold from phylum to genus level by mothur (v1.36.1) using the RDP reference database (trainset 1664) and to species level by SPINGO65 (v1.3) using the RDP reference database (v11.4) Alpha (a) and beta (β) diversity analyses were performed in QIIME on a rarefied OTU table. The sequences were aligned using the PyNast tool in Qllme to generate α-diversity indices, i.e., Shannon, Simpson, PD whole tree, Chao1, and Observed Species, and β-diversity indices, i.e., Bray-Curtis, Weighted UniFrac, and Unweighted UniFrac.

### Shallow shotgun analysis

Pre-processing of shotgun sequence data: Pre-processing of raw shotgun sequence reads was performed using a similar approach adopted by previous studies. To summarize, the reads were quality trimmed using Trimmomatic (with default parameters); followed by removing reads originating from the host genome bowtie2 with default parameters (with Mus musculus genome version 9 for mouse fecal metagenomes and human genome hg38 for donor fecal metagenomes).

Taxonomic, functional, and strain-wise profiling: The taxonomic and functional profiling of the metagenomes were performed using the humann2 pipeline with the clade-specific marker gene-based metaphlan2 as the taxonomic classifier. Pathway and gene-family abundances obtained using the UniProt mapping scheme were subsequently converted into the KEGG-specific mapping scheme using the legacy databases of humann2 (as described in Keohane et al. Microbiome and health implications for ethnic minorities after enforced lifestyle changes. Nat Med 1-7 (2020) doi:10.1038/s41591-020-0963-8*.).* Strain-wise variations were profiled using Strainphlan2. Inferred metabolite production and consumption profiles were obtained using a similar approach as adopted in Ghosh et al. (Adjusting for age improves identification of gut microbiome alterations in multiple diseases. eLife 9, 211 (2020*).).*

### RNA-Sequencing

RNA sequencing libraries were prepared by Genewiz with the Standard RNA-seq protocol for tumor samples and by GATC (Eurofins) for healthy control samples. Tumor samples were sequenced on an Illumina HiSeq instrument with 150-bp paired-end reads to an average depth of 29 million pairs of reads per sample. Healthy control samples were sequenced with 100-bp paired-end reads to an average depth of 45 million pairs of reads per sample. Raw data were deposited in Gene Expression Omnibus.

### RNAseq transcriptome analysis

Quality control checks of paired-end sequencing reads were performed using FastQC software (v0.11.5). The TrimGalore (v0.6.5) wrapper tool around Cutadapt (v1.15) and FastQC was used to apply quality and adapter trimming to FASTQ files. STAR (v2.7.3a) was used to align trimmed reads to the human genome (hg38) with the --quantMode GeneCounts option to output read counts per gene. The Bioconductor package EdgeR (v3.28.1) was applied in R (v3.6.3) to identify statistically significant differentially expressed genes between patient groups. To account for biological and technical variation, data was modeled as a negative binomial distribution using a generalization of the Poisson distribution model. The filterByExpr function was applied to remove lowly expressed genes. The data was normalized across library sizes, between samples using the trimmed mean of M-values (TMM) normalization method. Tagwise dispersions were estimated for the normalized dataset. Statistical tests were corrected for multiple testing using the Benjamini-Hochberg method in EdgeR. For the comparisons between tumors and healthy controls genes were considered significantly differentially expressed with an FDR adjusted p value < 0.1. For the comparisons between Pathogen and Lachnospiraceae enriched tumor genes were considered significantly differentially expressed with a p value < 0.05. Voom "variance modeling at the observational level" method within edgeR was used to output normalized read counts as LogCPM values. These were used to perform hierarchical clustering and to construct heatmaps in Gene Pattern's online server (v3.9.11), volcano plots in Gene Patterns Multiplot Studio (v1.5.2), to estimate the abundances of immune cell types in a mixed cell population with CIBERSORTx25,26 signature genes (LM22), and to perform Gene Set Enrichment Analysis (GSEA) (v4.1.0) with annotated HALLMARK genesets from the MSigDB (Molecular Signatures Database) collections (v6.2). Venn diagrams were constructed using InteractiVenn. Further statistical analysis of estimated abundances of immune cell types from CIBERSORTx involved students t-tests between patient groups within GraphPad Prism (v6).

### Statistical analysis

Statistical analysis, data visualization and machine learning based analyses were carried out in R statistical software package (v3.4.0). Principal Coordinate Analyses (PCoA) were performed using the dudi.pco function of the ade4 package (v1.7-15). Two-dimensional PCoA plots were created using the ggplot2 package (v2.2.1). Permutational multivariate analysis of variance (PERMANOVA) analyses to test for statistical difference in β-diversity, the gut microbiome profiles as well as the strain variations of the different species were performed using the adonis function from the vegan package (v2.5-6). Spearman Distances of the species abundances across samples and the species-specific strain-wise distances were provided as inputs to the adonis function. PERMANOVA analysis of the strain-wise variations for each species (obtained using Strainphlan) was performed in a time-point specific manner (separately for week 2 and week 5), after adjusting for the donor and abundance of the given species as confounders. Effect size calculations (Cohen's D) were performed using the effsize package (v0.8.1). We excluded from the analysis any bacterial taxa or metabolites that were not present in at least 50% of the samples of each group. Significant variations in α-diversity, taxa/gene relative abundance, metabolites abundance, and pathway coverages were assessed on mean values of the technical replicates using the Mann-Whitney U test for unpaired data or Wilcoxon signed-rank test for paired data. The Kruskal-Wallis test followed by Dunn's post hoc test with Benjamini-Hochberg p value adjustment for multiple testing was applied when comparing more than two experimental groups. The bar plots showing different taxonomic level classification were created using the ggplot2 package. Taxa below 1% sample abundance and the unclassified taxa were grouped into the "Other" category. P values from multiple comparisons were adjusted for the FDR using the Benjamini-Hochberg method (implemented in the p.adjust R function). Significance was assumed for adjusted p values ≤0.05, if not stated otherwise. Correlations between metabolite and taxa relative abundances were calculated using standard Spearman's rank correlation using the 'corr' function implemented in the 'psych' module of R and hierarchical clustering was computed using the hclust function in R (method "complete"). Features (that is taxa at various time-points and inferred metabolites) that showed significant Spearman correlations with tumor volumes (FDR < 0.1, obtained after p value adjustment using the Benjamini-Hochberg method) were visualized using the ggplot2 package.

### Machine-learning based analysis

For comparative validation on a global scale, we utilized the curatedMetagenomicData repository to six additional case-control datasets containing human fecal shotgun metagenome data from greater than 600 individuals consisting of CRC patients and controls (this was referred to as the 'Global Reference CRC cohort'). For comparing taxa abundances or inferred metabolite inferences within this Global Reference CRC cohort, we adopted a two-step procedure. First, for inter-dataset variability in the detection of various taxa, we performed across sample rank normalizations of taxa abundances separately within each dataset corresponding to the six studies. This limited the abundance range of each taxon from 0 to 1 uniformly for all the six studies. Subsequently, the rank normalized profiles for the six studies were combined and the comparison of rank normalized taxa abundances were compared between CRC patients and non-diseased individuals using Mann-Whitney U tests. Machine-learning based analyses consisting of evaluating the disease predictive ability of various markers in the Global Reference CRC cohort as well as within our dataset were performed using Random Forest approach (using the randomForest module of the R-programming interface). Iterative random forest classifiers built by taking repeated 50% subsets of 'test' and 'training' samples were obtained using the same methodology as used in Ghosh et al (Adjusting for age improves identification of gut microbiome alterations in multiple diseases. eLife 9, 211 (2020*)).*

### Flow cytometry

Spleens were harvested 19 days after MC-38 cancer cells injection and processed for flow cytometry analysis as previously described. Spleens were forced through a 70 µm cell strainer, and flow-through cells were collected for experiments. Cells were resuspended in 1x DPBS supplemented with 0.5% BSA and centrifuged at 300x g. The cell suspension was filtered through a 70 µm cell strainer, and red blood cells were lysed using red blood cell lysing buffer (Sigma-Aldrich) for 1 min at room temperature.

For flow cytometry staining, cells (1×106) were pre-incubated with purified anti-mouse CD16/CD32 (clone 2.4G2, Biolegend) for 10 min on ice, and then stained with the appropriate surface markers antibodies at 4°C for 30 min in the dark (see below for antibodies). Cell viability stain Zombie Red (BioLegend, San Diego, CA) was used to differentiate between dead and live cells. The stained populations were analyzed using a BD FACSCelesta^{™} flow cytometer (BD, USA) and FlowJo software (BD, v10). Antibodies were titrated for optimal staining. Antibodies used were: CD45-BV510 (Clone 30-F11, dil 1/100), CD274 (B7-H1, PD-L1)-BV605 (Clone 10F.9G2, dil 1/100), Ly6G-BV786 (clone 1A8, dil 1/100), CD11b-FITC (clone M1/70, dil 1/100), CD103-PE (Clone 2E7, dil 1/200), F4/80-PerCP-Cy5.5 (Clone BM9, dil 1/80), CD11c-APC (Clone N418, dil 1/200), Ly6C-AF700 (clone HK1.4, dil 1/200), CD3-FITC (Clone 17A2, dil 1/100), NKp46-PE (clone 29A1.4, dil 1/50), CD4-PerCP-Cy5.5 (Clone RM4-4, dil 1/200), CD279 (PD-1)-APC (clone 29F.1A12, dil 1/200), CD8-AF700 (Clone 53-6.7, dil 1/200), all from BioLegend; MHC II (I-A/I-E)-APC-eFluor 780 (Clone M5/114.15.2, dil 1/200) from eBioscience.

### Statistical analysis and reproducibility

Statistics for 16S rRNA gene sequencing, shotgun metagenomic sequencing, and human RNA-seq are described above. Other data were graphed and analyzed using GraphPad Prism 7 for Windows, and specific statistical methods used are indicated in the text or figure legends. Briefly, a two-tailed Mann-Whitney U test was used for comparison between two groups. For multi-group comparisons, one-way or two-way ANOVA followed by Dunn's multiple comparisons test was performed. Only statistically significant differences are indicated in the figures. For all statistical analyses: *, p <
0.05; **, p < 0.01; ***, p < 0.001; ****, p < 0.0001. Exact p values and statistical tests used for each panel are reported in the source data. Sample size of mice follows the 3 Rs (replace, reduce, and refine). Mice were randomly assigned to experimental groups and matched to the best age. No data were excluded from the analyses and details on experiment repetition are given in the respective figure legends. The investigators were not blinded to group allocation for mouse stool collection and euthanasia to avoid sample cross-contamination.

### Immunofluorescence staining, imaging and quantification

Methacarn-fixed paraffin sections of human CRCs were subjected to heat-mediated antigen retrieval (TE buffer, pH9) prior to blocking with 10% fetal bovine serum, 2% BSA, 0.02% fish skin gelatin, 0.05% TritonX100 (Sigma) and 0.05% Tween (Sigma). After 1h blocking at room temperature, primary antibodies were incubated overnight at 4°C, followed by 1h incubation at room temperature in secondary antibody.
The following primary antibodies were used: mouse anti-CD45 (Leica Biosystems, NCL-LLCA, 1/70), mouse anti-CD3 (Leica Biosystems CD3565LCE, 1/100), mouse anti-CD8 (Novus Biologicals, NBP2-32952, 1/200), mouse anti-CD15 (Cell Signalling, SSEA1 MC480 #4744S, 1/1500). For immunofluorescence, the following conjugated secondary antibodies were used: AlexaFluor555 donkey anti-mouse (all Invitrogen, purchased from ThermoFisher Scientific, 1/300). DAPI (Life Technologies) was used as a nuclear counterstain. Slides were mounted using ProLong Gold anti-fade reagent (Life Technologies). TissueFAXS Quantitative Imaging System (TissueGnostics, Vienna, Austria) was used to acquire images from the slides. The TissueFAXS uses a standard widefield epi-fluorescence Zeiss AXIO Observer.Z1 Inverted Microscope (with high efficiency fluorochrome specific DAPI, GFP, CFP, Cy3, and Cy5). Images were captured with a Hamamatsu ORCA-Flash 4.0 CMOS Camera. The entire slide (75 x 25 mm²) was scanned at low magnification using a 5x objective to identify the location of the tissue on the slide, followed by acquisition in multiple sequential tiles at 20× high magnification used for all downstream analysis. Image processing and analysis was performed using StrataQuest software version 6.0.1.145 (TissueGnostics, Vienna, Austria). Image processing included reconstruction of whole images and creation of *in silico* multiplexed images. Tissue cytometry and backgating into the tissue images were used for quantitation and visualization of the *in silico* data. In brief, several algorithms were used: to isolate cells by DAPI staining, to create a ring mask and identify non-nuclear staining starting from the centroid of the identified nucleus and stopping at the exterior of the biomarker, to identify the biomarker for the cell phenotype (CD45, CD3, CD8, CD15 stainings), allowing isolation of individual cells by a specific phenotype. Global standard measurements were computed for area (µm²), mean fluorescence intensity, perimeter (µm), compactness, and cell location (Cartesian coordinates). 2D dot scatterplots were created for each ROI containing the mean fluorescence intensity of one biomarker on each axis. Using the backgating algorithm, threshold cutoffs were manually positioned to include or exclude cell subpopulations.

### Quantification of immunostaining

To quantify immune cell infiltrate, images were analyzed using open-source ICY software plug-in Manual Counting and Spot Detector. Total number of cells (DAPI⁺) and positively stained cells for CD45, CD3, CD8, and CD15 were segmented in each ROI (region of interest).

*Isolation of strains of Lachnospiracaea CAG from Human fecal inoculum preparation* Fresh fecal or tumor samples were homogenised in pre-reduced PBS, and the homogenate was diluted and spread onto plates of different media. Plates were incubated under anaerobic conditions in an atmosphere of 90% N₂, 5% CO₂, and 5% H₂ at 37°C for up to 4 days. Isolates were picked and re-streaked to purity. Pure cultures were stored with glycerol 20% (v/v) at -80°C. DNA was extracted using the Qiagen DNeasy blood and tissue kit (Qiagen). The isolates were identified by PCR amplification of the 16S rRNA gene using the universal bacterial primers 27F2 and 1492R3 from Eurofin Genomics After Sanger sequencing of the 16S amplicon carried out by GATC Biotech Ltd (UK), reads were aligned in BLASTn to assign closest species match.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A composition comprising a pure culture of at least one bacterium, for use in the treatment or prevention of colorectal cancer in a subject in which the composition is administered to the colon of the subject, in which the bacterium is a human gut-derived bacterium selected from: *Coprococcus comes; Ruminococcus lactaris; Bifidobacterium longum; Bifidobacterium pseudocatenulatum;* and *Bacteroides finegoldii.*

2. A composition according to Claim 1, for use of Claim 1, in which the composition comprises a consortium of bacteria comprising at least two bacterial strains selected from: *Coprococcus comes; Ruminococcus lactaris; Bifidobacterium longum; Bifidobacterium pseudocatenulatum;* and *Bacteroides finegoldii,* in which each bacterium in the consortium of bacteria is provided as a pure culture.

3. A composition according to Claim 1, for use of Claim 1, in which the composition comprises a consortium of bacteria comprising at least three bacterial strains selected from: *Coprococcus comes; Ruminococcus lactaris; Bifidobacterium longum; Bifidobacterium pseudocatenulatum;* and *Bacteroides finegoldii,* in which each bacterium in the consortium of bacteria is provided as a pure culture.

4. A composition according to Claim 1, for use of Claim 1, in which the composition comprises a consortium of bacteria comprising at least four bacterial strains selected from: *Coprococcus comes; Ruminococcus lactaris; Bifidobacterium longum; Bifidobacterium pseudocatenulatum;* and *Bacteroides finegoldii,* in which each bacterium in the consortium of bacteria is provided as a pure culture.

5. A composition according to any of Claims 2 to 4, for use of Claim 1, in which the consortium of bacteria comprises *Coprococcus comes.*

6. A composition according to any of Claims 2 to 4, for use of Claim 1, in which the consortium of bacteria comprises *Ruminococcus lactaris*

7. A composition according to any of Claims 1 to 6, for use of Claim 1, in which the or each bacterium is derived from the microbiota of a subject that is colorectal cancer negative.

8. A composition according to any of Claims 1 to 7, for use of Claim 1, in which the method is a method of inhibiting or slowing growth or reducing a volume of a colorectal tumour in the subject.

9. A composition according to any of Claims 1 to 7, for use of Claim 1, in which the subject is at risk of developing colorectal cancer and the method is a method of preventing, slowing or inhibiting development of colorectal cancer in the subject.

10. A composition according to any of Claims 1 to 7, for use of Claim 1, 8 or 9, in which the method comprises administering about 10³ to 10¹² cfu of the or each bacterium to the subject.

11. A composition according to any of Claims 1 to 7 and 10, for use of Claim 1, 8 or 9, in which the method additionally comprises administering to the subject an anti-microbial agent targeted against a bacterium selected from the group consisting of:
*Clostridium hathewayi;*
*Flavonifractor plautii;*
*Bacteroides fragilis;*
*Bacteroides salyersiae;* and
*Clostridium bolteae.*

12. A method of formulating a pharmaceutical composition comprising the steps of:
isolating at least one strain of the *Lachnospiraceae* CAG, which is selected from *Coprococcus comes; Ruminococcus lactaris; Bifidobacterium longum; Bifidobacterium pseudocatenulatum;* and *Bacteroides finegoldii;*
culturing the isolated strain to provide a pure culture of the strain; and
combining the pure culture of the strain with a pharmaceutical excipient to provide the pharmaceutical composition.

13. A method according to Claim 12, comprising:
isolating a plurality of strains of the *Lachnospiraceae* CAG selected from *Coprococcus comes; Ruminococcus lactaris; Bifidobacterium longum; Bifidobacterium pseudocatenulatum;* and *Bacteroides finegoldii;*
culturing the isolated strains to provide a pure culture of each strain; and
combining the pure cultures with a pharmaceutical excipient to provide the pharmaceutical composition.

14. A pharmaceutical composition obtained by a method Claim 13 or 14,

15. A pharmaceutical composition of Claim 14, for use in a method of treating or preventing colorectal cancer in a subject, in which the composition is administered to the distal colon of the subject.
